# EUROPEAN PATENT APPLICATION

(11) **EP 3 424 471 A1**
(43) Date of publication of application: **09.01.2019**
(21) Application number: 17759969.3
(22) Date of filing: 28.02.2017
(51) Int. Cl.: A61F 5/451

(54) **STOOL RECEPTACLE OF STOOL PROCESSING DEVICE FOR USE IN NURSING CARE**

(30) Priority: 29.02.2016 JP 2016001221 U; 14.02.2017 JP 2017024824
(71) Applicant: Libertysolution Co., Ltd., Matsue-shi, Shimane 690-0048 (JP)
(72) Inventor: FURUTA Miyuki, Matsue-shi Shimane 690-0048 (JP)
(74) Representative: Vossius & Partner Patentanwälte Rechtsanwälte mbB
(86) International application number: PCT/JP2017/007691
(87) International publication number: WO 2017/150503

(57) **Abstract**

The present invention addresses the problem of providing a stool receptacle of a stool processing device that is for use in nursing care and that has significantly improved leakage prevention. The problem is addressed by a stool receptacle comprising: a stool receiving cup (5) having a bottom plate part (2) that is positioned at a buttocks-side of a wearer, a front plate part (3) that rises from the bottom plate part and that has a U-shaped axial cross-section, and a cap part (4) that is joined to and integrated with an upper end of the front plate part and that is positioned at an abdomen-side of the wearer; and an attaching and fixing member (12) that fixes the stool receiving cup to the crotch of the wearer. The bottom plate part has a pan-shaped storage part (2A) that receives discharged stool, and the cap part comprises a sheet (4B) that has a larger area than the bottom plate part and that faces the bottom plate part. The sheet is configured to be so adhesive as to be attachable to the skin of the wearer without allowing any liquid to leak, and to be thus capable of being brought into close contact with the abdomen of the wearer. The attaching and fixing member is formed in the shape of a strip having a sufficient length to wrap around the abdomen of the wearer from the buttocks.

## Description

### Technical Field:

This invention relates to a stool receptacle of a stool processing device for use in nursing care.

### Background Arts:

The number of persons to be cared such as bedridden aged persons and severely physically handicapped persons who are incapable of defecating and/or urinating alone goes on increasing year after year.

In this connection, various types of stool processing devices for use in nursing care to automatically perform processing on defecation and/or urination of such persons to be cared have been devised and put into practical use.

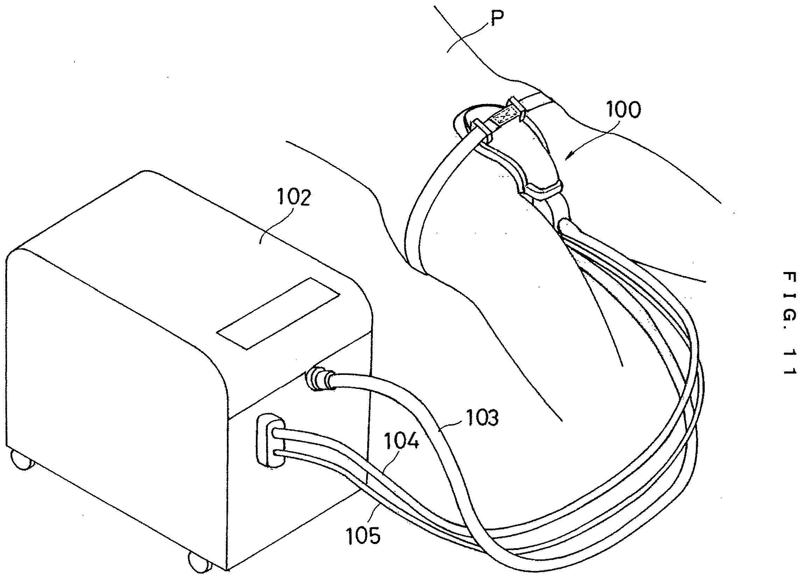

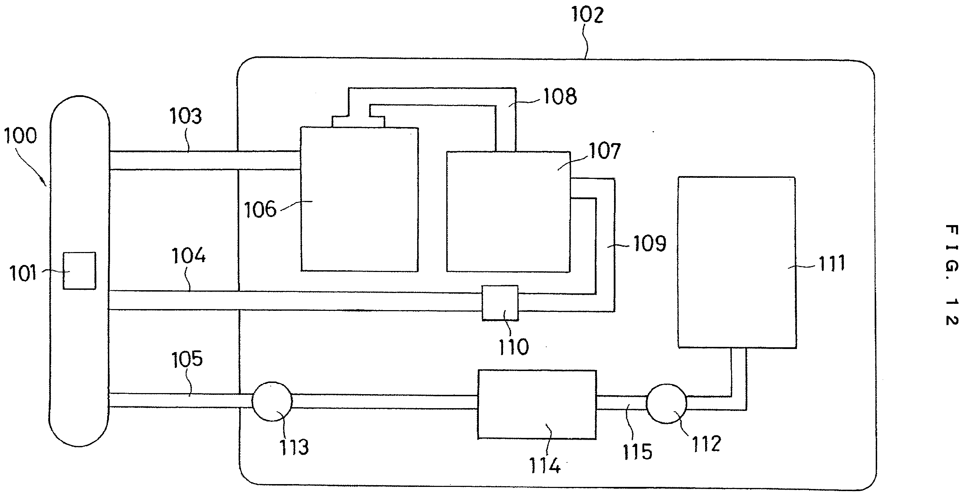

These stool processing devices for use in nursing care each have a basic configuration generally as shown in FIGS. 11 and 12.

Referring to FIGS. 11 and 12, reference numeral 100 designates a stool receptacle that is applied to the crotch of a person to be cared and in which a pan-shaped storage part of a bottom plate part is provided with a stool detection sensor 101, while a front plate part is provided with a filth suction port, a washing water jetting port and an air blow-out port in this order from a lower side. Reference numeral 102 designates an equipment housing box, and reference numerals 103, 104 and 105 respectively designate hoses through which the stool receptacle 100 and each equipment housed in the equipment housing box 102 are connected together, wherein the hose 103 is for suction of filth, the hose 104 is for sending of air and the hose 105 is for feed of washing water.

Reference numeral 106 designates a filth tank that is provided inside the equipment housing box 102 and into which a filth consisting of a mixture of washing water and discharged stool and urine is sucked together with air from the filth suction port of the stool receptacle 100 through the hose 103 and is then stored. Reference numeral 107 designates a suction motor that is connected to the filth tank 106 by using a suction circulation pipe 108 to generate suction force for suction from the filth tank 106 with a pressure differential between atmospheric pressure in the suction circulation pipe 108 and atmospheric pressure in the filth tank 106 in condition where the atmospheric pressure in the suction circulation pipe 108 is reduced lower than the atmospheric pressure in the filth tank 106. Further, the suction motor 107 sucks air in the filth tank 106 and then delivers the air to a suction circulation pipe 109 having an air cleaning filter 110 in the middle, followed by sending the air from the suction circulation pipe 109 to the connection hose 104.

Reference numeral 111 designates a washing water tank to which a washing water hose 115 having, in the middle, water feed pumps 112 and 113 and a hot water tank 114 equipped with a heater is connected to connect the washing water hose 115 to the washing water jetting port of the stool receptacle 100 through the connection hose 105.

An operation of the stool processing device for use in nursing care is as follows.

First of all, the stool receptacle 100 is attached to the crotch of a person P to be cared. Then, after the lapse of a fixed time since the stool detection sensor 101 of the stool receptacle 100 detects discharged stool, the water feed pump 113 is actuated to feed appropriately heated washing water from the hot water tank 114 to the stool receptacle 10 through the washing water hose 115 and the connection hose 105. By so doing, the washing water is jetted from the washing water jetting port toward the anus and so on for washing, and thereafter, the stool is led into the filth suction port together with filthy water resulting from washing. At the same time, the suction motor 107 is actuated to suck the stool together with the filthy water resulting from washing from the filth suction port of the stool receptacle 100 into the filth tank 106 through the connection hose 103. Meanwhile, the air in the filth tank 106 is sent to the stool receptacle 100 through the suction circulation pipe 109 and the connection hose 104 after being cleaned by passing through the air cleaning filter 110, and is then blown out from the air blow-out port to dry the crotch of the person to be cared.

The present invention relates to the stool receptacle 100 of the stool processing device for use in nursing care.

Various types of the arts relating to the stool receptacle 100 have been devised and put into practical use even so far (see a patent document 1 below, for instance).

However, any conventional stool receptacle is low in close contactness between the stool receptacle and the crotch of the person to be cared, and therefore, has frequently brought about such situations as leakage of part of the washing water in jetting and/or leakage of the filth and/or filthy water at the occurrence of body movements of the person to be cared who is in a posture of lying on one's side.

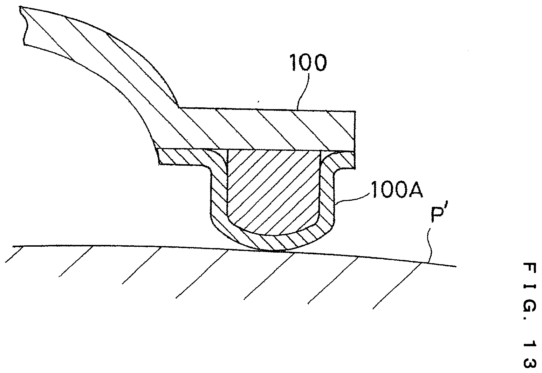

The reason that the close contactness with the crotch of the person to be cared is low in the case of the conventional stool receptacle is because of the configuration of a leakage preventive structure for each of an opening part of a pan-shaped storage part of a bottom plate part, a front plate part that rises from the bottom plate part and a cap part that is provided at the top of the front plate part. Namely, the leakage preventive structure for each of the above parts has a cushioning protrusion 100A as being like an embankment, as shown in FIG. 13, and is for pressing the protrusion 100A against the skin P' of the person to be cared, with pressure applied from the external.

However, only pressing of the protrusion 100A in this manner results in a condition in which the protrusion 100A is merely in abutment with the skin P' of the person to be cared and in which an axial cross-section thereof is in an extremely thin shape, and consequently, contact with the skin P' of the person to be cared would be confined only to an extremely small surface area. For that reason, there is a possibility also that a fine clearance would be caused by positional shift of a contacting portion when the person to be cared turns oneself over while lying.

### Prior Art Documents

### Patent Document

Patent document 1: Japanese Unexamined Patent Application Publication No. 2016-22367

### Summary of the Invention:

### Problems to be solved by the Invention:

In view of the foregoing, it is an object of the present invention to provide a stool receptacle of a stool processing device that is for use in nursing care and that has significantly improved leakage prevention on the grounds that a structure for attaching by utilization of adhesive force is provided as a leakage preventive structure, in place of a conventional structure for pressing a protrusion, so as to make it possible to achieve close contact entirely over a large surface area particularly at an abdomen side in a state of being free from any clearance without causing any positional shift.

### Means for solving the Problems:

To solve the above problems, the present invention provides a stool receptacle of a stool processing device that is for use in nursing care and that comprises: a stool receiving cup having a bottom plate part that is positioned at a buttocks-side of a wearer, a front plate part that rises from the bottom plate part and that has a U-shaped axial cross-section and a cap part that is joined to and integrated with an upper end of the front plate part and that is positioned at an abdomen-side of the wearer; and an attaching and fixing member that fixes the stool receiving cup to the crotch of the wearer, wherein the bottom plate part has a pan-shaped storage part that receives discharged stool, wherein the cap part includes a sheet that has a larger area than the bottom plate part and that faces the bottom plate part, wherein the sheet is configured to be so adhesive as to be attachable to the skin of the wearer without allowing any liquid to leak, and to be thus capable of being brought into close contact with the abdomen of the wearer, and wherein the attaching and fixing member is formed in the shape of a strip having a sufficient length to wrap around the abdomen of the wearer from the buttocks.

It is allowable also that the attaching and fixing member has, on the longitudinal center, a pad part that has a larger area than the buttocks of the wearer.

It is allowable also that the pad part has elasticity.

It is allowable also that the attaching and fixing member has, at both longitudinal ends, a securing member that is capable of changing a securing position.

It is allowable also that the pad part has a recess part, thus permitting the bottom plate part of the stool receiving cup to be fixed to the inside of the recess part in a freely attachable/detachable manner.

It is allowable also that the pad part has a housing part that is formed in a flexibly deformable bag shape and a filling member that is housed in the housing part and that has elasticity.

It is allowable also that the filling member is provided in a plural number, the plurality of filling members being respectively in the form of plate-like members that are formed in separate bodies and that have different elasticity, the plurality of plate-like members being housed in the housing part in a stacked state.

It is allowable also that the sheet and the attaching and fixing member are formed in separate bodies.

It is allowable also that the attaching and fixing member in a state of being spread has a trapezoidal shape of which one side positioned at the foot side of the wearer forms a short side, while the other side positioned at the head side of the wearer forms a long side parallel to the short side.

It is allowable also that the cap part has an outwardly swollen and dish-shaped bulge part on the center, the bulge part being configured so as to have a plurality of small ventilation holes at the distal end in an extension direction.

### Effects of the Invention:

According to the present invention having the above configuration, the structure for attaching by utilization of adhesive force is provided as the leakage preventive structure, in place of the conventional structure for pressing the cushioning protrusion. By so doing, the close contact is made achievable entirely over the large surface area particularly at the abdomen side in the state of being free from any clearance without causing any positional shift, resulting in the significant improvement in leakage prevention.

Further, because the structure for attaching by utilization of adhesive force is provided, and besides, the cap part for covering the abdomen of the wearer has a large area, an attaching position can be also appropriately changed in accordance with a wearer's physical constitution.

### Brief Description of the Drawings:

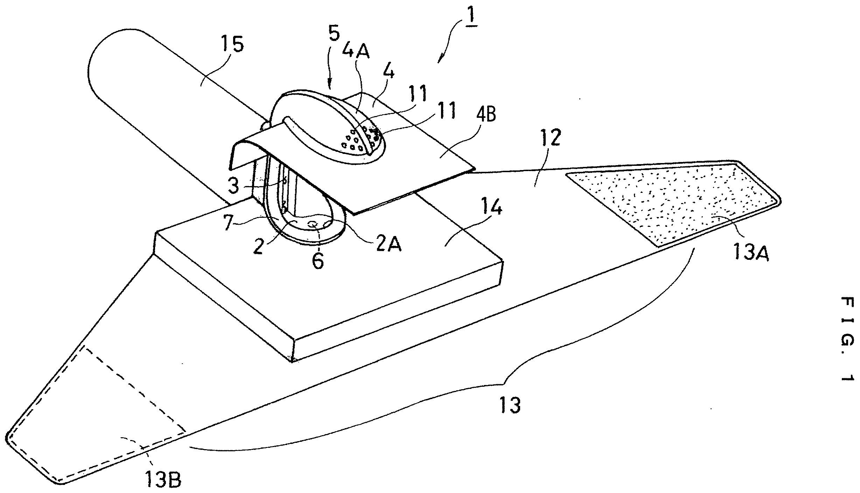

FIG. 1 is a perspective view of a stool receptacle of a stool processing device for use in nursing care according to an embodiment of the present invention.

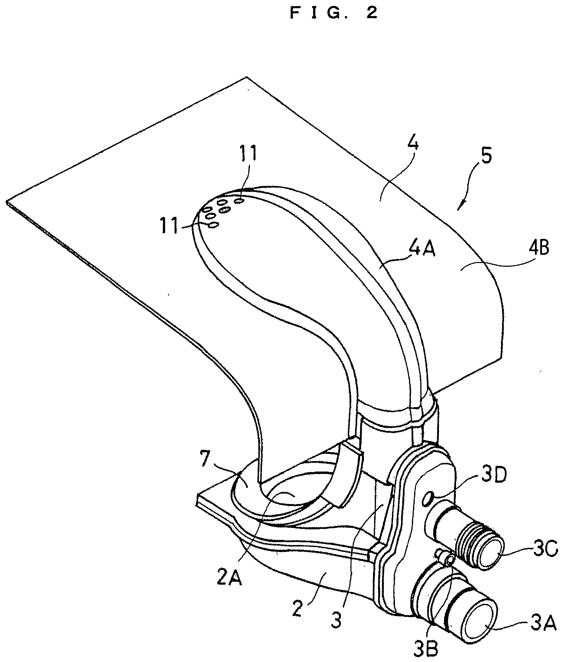

FIG. 2 is a perspective view of a stool receiving cup as viewed from a front surface side thereof.

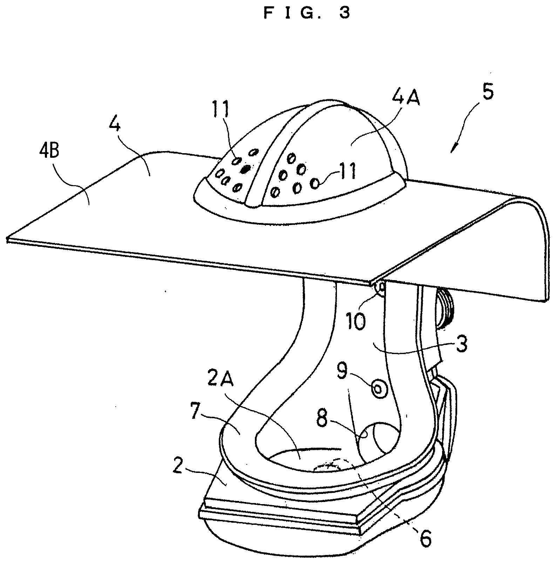

FIG. 3 is a perspective view of the stool receiving cup as viewed from a rear surface side thereof.

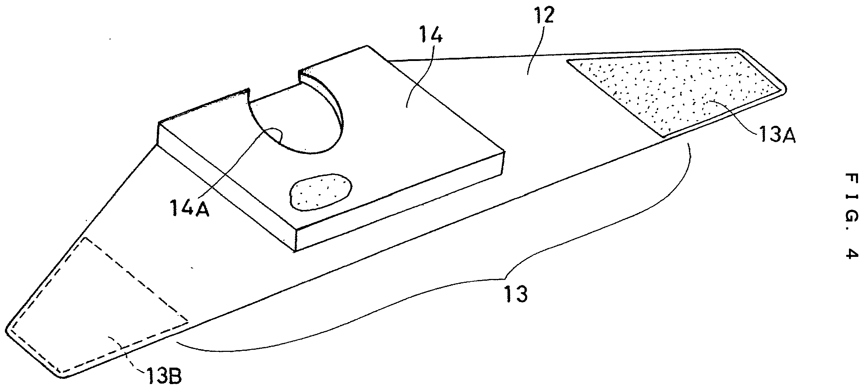

FIG. 4 is a partially cutaway view in perspective of an attaching and fixing member.
FIG. 5 is an illustration for explaining an attaching procedure.
FIG. 6 is a partially enlarged sectional view of a state of being attached to the skin of a wearer.
FIG. 7 is a sectional view showing a sheet constitution.
FIG. 8 is a perspective view showing a state of the stool receiving cup when being stored.
FIG. 9 is a plan view showing the attaching and fixing member according to another embodiment of the present invention.
FIG. 10 is a sectional view taken on line A-A in FIG. 9.
FIG. 11 is a schematic perspective view of a stool processing device for use in nursing care in the prior art.
FIG. 12 is a schematic block diagram of the stool processing device for use in nursing care in the prior art.
FIG. 13 is an illustration for explaining a leakage preventive structure of a conventional stool receptacle.

### Mode for Embodying the Invention:

Hereinafter will now be described an embodiment of the present invention with reference to the attached drawings.

Referring to the drawings, reference numeral 1 designates a stool receptacle of a stool processing device for use in nursing care. The illustrated stool receptacle 1 has the following configuration.

The stool receptacle 1 comprises a stool receiving cup 5 having a bottom plate part 2, a front plate part 3 that is formed integrally with the bottom plate part 2 by resin, that rises from the bottom plate part 2 and that has a U-shaped axial cross-section and a cap part 4 that is joined to and integrated with an upper end of the front plate part 3, and an attaching and fixing member 12 (described later) that fixes the stool receiving cup 5 to the crotch of a wearer.

The bottom plate part 2 of the stool receiving cup 5 has a pan-shaped storage part 2A that receives discharged stool, and the pan-shaped storage part 2A is provided with a stool detection sensor 6. Further, a surface ranging from an opening part of the pan-shaped storage part 2A to an edge of the front plate part 3 is applied with leakage preventive silicone rubber 7 that is appropriately so adhesive as to be attachable to the skin of the wearer without allowing any liquid to leak. It is noted that such application of the silicone rubber 7 may be by a suitable means such as bonding of strip-shaped silicone rubber with an adhesive or by thermal fusing, and thick-coating and curing of molten silicone rubber. It is noted also that the silicone rubber 7 is preferably formed in the shape of a strip having a flat surface and a proper width so as to be capable of being brought into contact with the skin of the wearer by a large surface area. It is noted also that the degree of adhesiveness may be enough the same as that to which the silicone rubber can be softly attached to the skin of the wearer without allowing any liquid to leak, whereas use of excessively high adhesive silicone rubber would give pains to the wearer because such silicone rubber is liable to strongly pull the skin of the wearer when released.

The cap part 4 joined to and integrated with the upper end of the front plate part 3 having a filth suction port 8, a washing water jetting port 9 and an air blow-out port 10 is configured such that the surface facing the bottom plate part 2 is constituted of a silicone rubber sheet 4B that is appropriately so adhesive as to be attachable to the skin of the wearer without allowing any liquid to leak and that has a larger area than the bottom plate part 2. And, the cap part 4 has, on the center, an outwardly swollen and elongate dish-shaped bulge part 4A that is formed so as to be continuous from the front plate part 3, and the bulge part 4A has a plurality of small ventilation holes 11 at the distal end in an extension direction. It is noted that the small ventilation holes 11 are to prevent the bulge part 4A from being contracted with negative pressure that is generated when suction of the filth in the pan-shaped storage part 2A is performed.

In order that the surface of the silicone rubber 7 and the silicone rubber sheet 4B surface facing the bottom plate part 2 in the cap part 4 is made adhesive as described the above, use of a suitable means such as application of an acrylic adhesive may be made. Alternatively, self-adhesive silicone rubber may be used as well, in place of such application of the acrylic adhesive. In this case, the silicone rubber sheet 4B of the cap part 4 is configured such that a polyethylene protective film is attached to the opposite side surface of the surface facing the bottom plate part 2, that is, an outer surface of the sheet. It is noted that the protective film is not required in the case where a single-sided self-adhesive silicone rubber sheet is employed.

A filth-suction hose connection port 3A, a washing water-feed hose connection port 3B, an air-sending hose connection port 3C and a sensor cord connection port 3D are provided at the outer surface side of the front plate part 3.

The attaching and fixing member 12 is formed of a flexible material such as cloth into the shape of a strip having a sufficient length to wrap around the abdomen of the wearer from the buttocks, and has, at both longitudinal ends, an appropriate securing member 13 that is capable of changing a securing position. The attaching and fixing member 12 further has, on the longitudinal center, an appropriately elastic pad part 14 that is wider than the buttocks of the wearer, and the pad part 14 has a recess part 14A in the center of the front end, thus permitting the bottom plate part 2 of the stool receiving cup 5 to be fixed to the inside of the recess part 14A in a freely attachable/detachable manner.

It is noted that a hook-and-loop fastener is preferable as the securing member 13, and a hook-side hook-and-loop fastener 13A is attached by sewing to one longitudinal end side of the attaching and fixing member 12, while a loop-side hook-and-loop fastener 13B is attached by sewing to the other longitudinal end side thereof. It is noted also that use of the hook-and-loop fastener is preferable as a means of fixing the bottom plate part 2 of the stool receiving cup 5 to the inside of the recess part 14A of the pad part 14. And, the attaching and fixing member 12 and the stool receiving cup 5 are capable of being freely separated independently of each other and can be thus cleaned and/or replaced in an independently separated state. In addition, reference numeral 15 in the drawings designates an auxiliary cover that wraps the connection hoses collectively.

Next will be described an operation of the present embodiment.

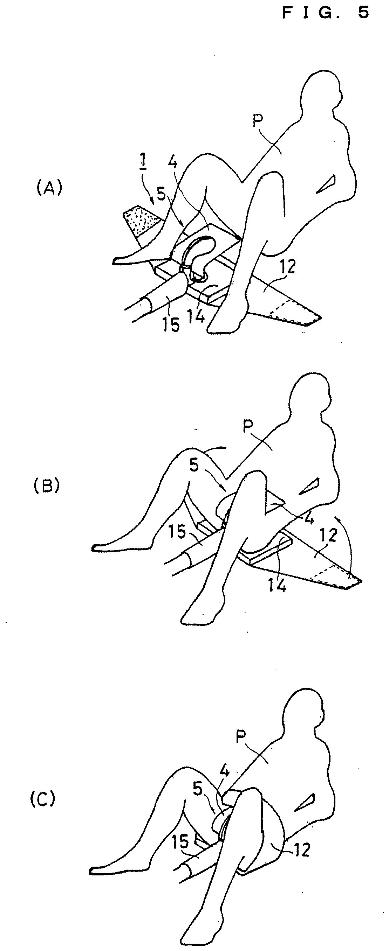

In attaching, the procedure shown in FIG. 5 is followed. It is noted that the pubic hair of a wearer P is preferable to be removed by shaving before attaching. First of all, as shown in FIG. 5(A), the stool receiving cup 5 is pushed upwardly to an upper-side position of the waist of the wearer P. At this time, the pan-shaped storage part 2A should be positioned right under the anus of the wearer. Next, as shown in FIG. 5(B), after setting the cap part 4 so as to wrap around the genitals of the wearer, the cap part 4 is fitted to the lower abdomen of the wearer. It is noted that in fitting, the adhesive force is utilized. Finally, as shown in FIG. 5(C), the attaching and fixing member 12 is wound around the abdomen of the wearer from the buttocks to fix the stool receiving cup 5 in position. And then after that, each equipment of the stool processing device for use in nursing care is actuated to perform washing, suction of the filth and sending of the air as described the above when the discharged stool in the pan-shaped storage part 2A is detected.

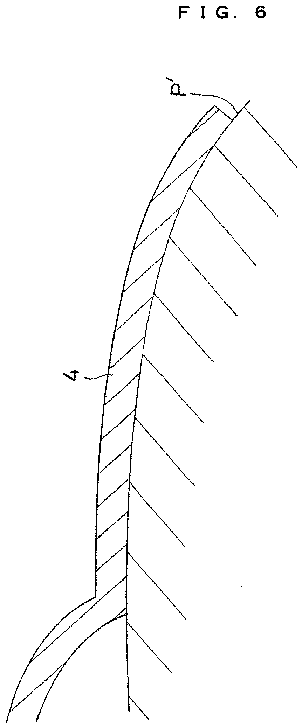

According to the present embodiment as described the above, the structure for attaching by utilization of adhesive force is provided as the leakage preventive structure, in place of the conventional structure for pressing the cushioning protrusion. Namely, the surface ranging from the opening part of the pan-shaped storage part 2A to the edge of the front plate part 3 is applied with the leakage preventing silicone rubber 7 that is appropriately so adhesive as to be attachable to the skin of the wearer without allowing any liquid to leak, while the cap part 4 is configured such that the surface facing the bottom plate part 2 is constituted of the silicone rubber sheet 4B that is appropriately so adhesive as to be attachable to the skin of the wearer without allowing any liquid to leak and that has a larger area than the bottom plate part. By so doing, the close contact is made achievable entirely over the large surface area particularly at the abdomen side in the state of being free from any clearance without causing any positional shift, as shown in FIG. 6, resulting in the significant improvement in leakage prevention. Further, because the structure for attaching by utilization of adhesive force is provided, and besides, the cap part 4 for covering the abdomen of the wearer has the large area, an attaching position can be also appropriately changed in accordance with a wearer's physical constitution. Furthermore, the silicone rubber is excellent in oil resistance and water resistance so as to be less susceptible to deterioration even at sweating, also has satisfactory compatibility with the skin of the wearer so as to hardly give rise to stuffiness caused by sweating and/or rash caused by contact with sweat, and can be easily washed and dried.

It is noted that a material of the sheet 4B forming the cap part 4 is not limited to the silicone rubber and therefore, may be other synthetic resins as long as being appropriately so adhesive as to be attachable to the skin of the wearer without allowing any liquid to leak. By the way, according to the present embodiment, a part of the sheet 4B and the bulge part 4A are formed integrally by the same material.

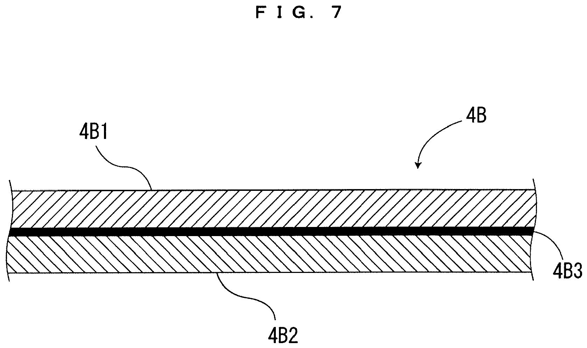

FIG. 7 is a sectional view showing a sheet constitution. The sheet 4B has a sheet body 4B1 that is formed integrally with the bulge part 4A and a functional sheet 4B2 that is attached entirely to the surface of the sheet body 4B1 at the side contacting the skin of the wearer.

The functional sheet 4B2 is constituted with silicone rubber or other synthetic resin that may satisfy the conditions for adhesiveness as described the above. The functional sheet 4B2 is integrally attached to the sheet body 4B1 with an adhesive 4B3 or by thermo-compression bonding. The above constitution of the sheet 4B enables a material for the most part of the sheet 4B to be freely selected, while holding the close contactness with the wearer, and accordingly, the sheet 4B can be formed integrally with the bulge part 4A by the same material as well.

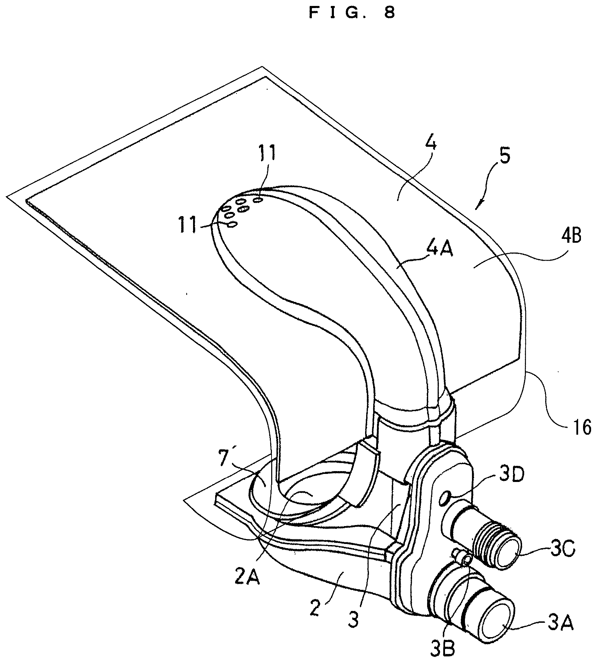

As shown in FIG. 8, when the stool receiving cap 5 is being stored, both the sheet 4B surface facing the bottom plate part 2 and the surface of the silicone rubber 7 may be covered with a protective film 16 that is elastically deformed into a curved shape along an inner surface side of the stool receiving cup 5 that is formed to have a U-shaped side view. By so doing, the stool receiving cap can be kept in good and sanitary storage condition.

Further, the stool receiving cup 5 shown in FIG. 8 is configured such that a wetting preventive member 7' formed in the same shape as the silicone rubber 7 and constituted with a material other than the silicone rubber is provided from the opening part of the pan-shaped storage part 2A to the edge of the front plate part 3, in place of the silicone rubber 7. The wetting preventive member 7' is subjected to surface treatment to be made so adhesive as to be attachable to the skin of the wearer without allowing any liquid to leak, or alternatively, is constituted with a material such as synthetic resin that may satisfy such conditions for adhesiveness.

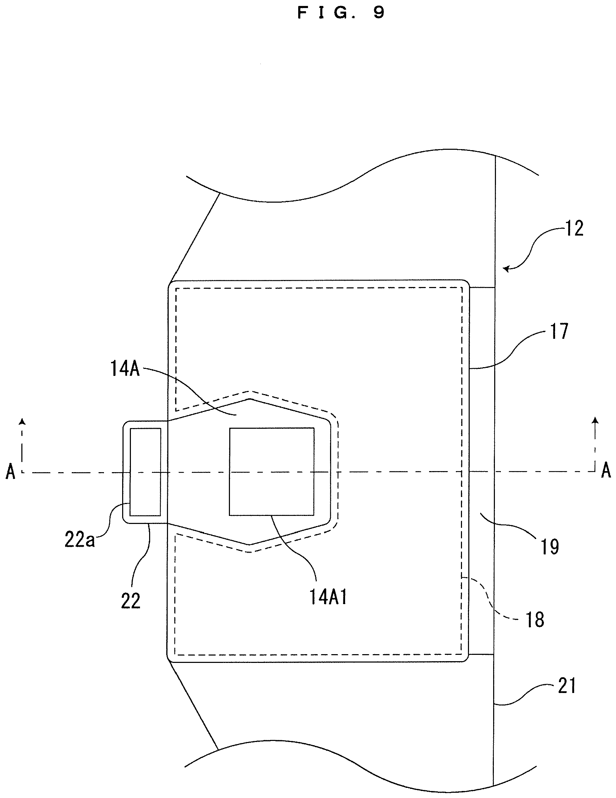

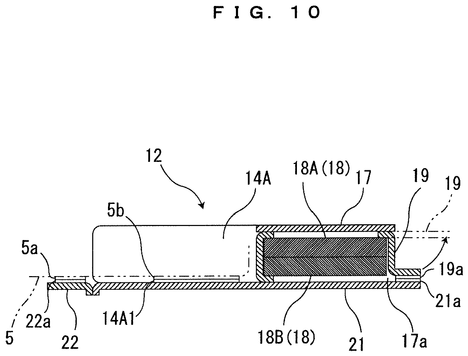

Next will be described another embodiment of the present invention in relation to parts different than the above embodiment with reference to FIGS. 9 and 10.

FIG. 9 is a plan view of the attaching and fixing member according to another embodiment of the present invention, and FIG. 10 is a sectional view taken on line A-A in FIG. 9. The attaching and fixing member 12 shown in FIGS. 9 and 10 is configured such that the pad part 14 is constituted of a housing part 17 that is formed in a flexibly deformable bag shape and a filling member 18 that is housed in the housing part 17 and that has elasticity.

The housing part 17 forms a housing space that is formed concurrently with formation of the strip-shaped attaching and fixing member 12 that is constituted by sewing the pieces of flexibly deformable cloth together. The housing part 12 has an opening part 17a at one end that is positioned at the side closer to the head of the wearer, the opening part 17a being opened or closed by a cap piece part 19 that is formed integrally with the housing part 12. Specifically, the opening part 17a is opened or closed by engagement or disengagement between a hook-and-loop fastener (an engagement part) 19a that is provided at an inner surface side of the cap piece part 19 and a hook-and-loop fastener 21a that is provided on a body 21 that forms a portion of the attaching and fixing member 12 other than the housing part 12.

The filling member 18 is provided in a plural number in the single housing part 12. The plurality of filling members 18 are each formed in a plate shape. Specifically, one filling member 18 forms a first filling member (a plate-like member) 18A that is constituted with a material such as silicone rubber and synthetic resin and that has the adjusted elasticity and/or flexibility obtained by formation of a plurality of recess parts or holes. The other filling member 18 forms a second filling member (a plate-like member) 18B that is constituted by forming a synthetic resin-made material into a sponge-like shape.

The plurality of plate-like members 18A and 18B having different elasticity and/or flexibility in this manner are housed in the housing part 17 in a stacked state to form the pad part 14. By the way, according to the illustrated embodiment, the first filling member 18A and the second filling member 18B are stacked in a predetermined order such that the first filling member 18A is positioned closer to the skin of the wearer than the second filling member 18B.

A bottom surface of the recess part 14A of the attaching and fixing member 12 and an extension part 22 extended from the bottom surface of the recess part in the direction away from the opening part 17a are respectively provided with hook-and-loop fasteners (engagement parts) 14A1 and 22a. On the other hand, stool receiving cup 5 portions housed in the recess part 14A in a fitted state are respectively provided with hook-and-loop fasteners (engagement parts) 5a and 5b that are engaged with the hook-and-loop fasteners (the engagement parts) 14A1 and 22a in a disengageable manner.

And, the stool receiving cap 5 is attached to or detached from the attaching and fixing member 12 by engagement or disengagement between the hook-and loop fasteners 5a and 5b and those 14A1 and 22a.

### Explanation of Reference Numerals:

- 1:: Stool receptacle
- 2:: Bottom plate part
- 2A:: Pan-shaped storage part
- 3:: Front plate part
- 3A:: Filth-suction hose connection port
- 3B:: Washing water-feed hose connection port
- 3C:: Air-sending hose connection port
- 4:: Cap part
- 4A:: Bulge part
- 4B:: Sheet
- 5:: Stool receiving cup
- 5a:: Hook-and-loop fastener (Engagement part)
- 5b:: Hook-and-loop fastener (Engagement part)
- 6:: Stool detection sensor
- 7:: Leakage preventive silicone rubber (Silicone rubber)
- 7':: Wetting preventive member
- 8:: Filth suction port
- 9:: Washing water jetting port
- 10:: Air blow-out port
- 11:: Small ventilation hole
- 12:: Attaching and fixing member
- 13:: Securing member
- 14:: Pad part
- 14A:: Recess part
- 14A1:: Hook-and-loop fastener (Engagement part)
- 16:: Protective film
- 17:: Housing part
- 18:: Filling member
- 18A:: First filling member (Plate-like member)
- 18B:: Second filling member (Plate-like member)
- 19:: Cap piece part
- 19a:: Hook-and-loop fastener (Engagement part)
- 21:: Body
- 21a:: Hook-and-loop fastener (Engagement part)
- 22: Extension part
- 22a:: Hook-and-loop fastener (Engagement part)

## Claims

1. A stool receptacle of a stool processing device that is for use in nursing care and comprises:
a stool receiving cup having a bottom plate part that is positioned at a buttocks-side of a wearer, a front plate part that rises from the bottom plate part and that has a U-shaped axial cross-section, and a cap part that is joined to and integrated with an upper end of the front plate part and that is positioned at an abdomen-side of the wearer; and
an attaching and fixing member that fixes said stool receiving cup to the crotch of the wearer;
wherein said bottom plate part has a pan-shaped storage part that receives discharged stool;
wherein said cap part includes a sheet that has a larger area than said bottom plate part and that faces said bottom plate part;
wherein said sheet is configured to be so adhesive as to be attachable to the skin of the wearer without allowing any liquid to leak and to be thus capable of being brought into close contact with the abdomen of the wearer; and
wherein said attaching and fixing member is formed in the shape of a strip having a sufficient length to wrap around the abdomen of the wearer from the buttocks.

2. The stool receptacle of the stool processing device according to claim 1, wherein said attaching and fixing member has, on the longitudinal center, a pad part that has a wider area than the buttocks of the wearer.

3. The stool receptacle of the stool processing device according to claim 2, wherein said pad part has elasticity.

4. The stool receptacle of the stool processing device according to any one of claims 1 to 3, wherein said attaching and fixing member has, at both longitudinal ends, a securing member that is capable of changing a securing position.

5. The stool receptacle of the stool processing device according to any one of claims 2 to 4, wherein said pad part has a recess part, thus permitting the bottom plate part of said stool receiving cup to be fixed to the inside of the recess part in a freely attachable/detachable manner.

6. The stool receptacle of the stool processing device according to any one of claims 2 to 5, wherein said pad part has a housing part that is formed in a flexibly deformable bag shape and a filling member that is housed in the housing part and that has elasticity.

7. The stool receptacle of the stool processing device according to claim 6, wherein said filling member is provided in a plural number, the plurality of filling members being respectively in the form of plate-like members that are formed in separate bodies and that have different elasticity, the plurality of plate-like members being housed in said housing part in a stacked state.

8. The stool receptacle of the stool processing device according to any one of claims 1 to 7, wherein said sheet and said attaching and fixing member are formed in separate bodies.

9. The stool receptacle of the stool processing device according to any one of claims 1 to 8, wherein said attaching and fixing member in a state of being spread has a trapezoidal shape of which one side positioned at the foot side of the wearer forms a short side, while the other side positioned at the head side of the wearer forms a long side parallel to the short side.

10. The stool receptacle of the stool processing device according to any one of claims 1 to 9, wherein said cap part has an outwardly swollen and dish-shaped bulge part on the center, the bulge part being configured so as to have a plurality of small ventilation holes at the distal end in an extension direction.
